# EUROPEAN PATENT SPECIFICATION

(11) **EP 1 815 823 B1**
(45) Date of publication and mention of the grant of the patent: **03.12.2008**
(21) Application number: 07250193.5
(22) Date of filing: 18.01.2007
(51) Int. Cl.: A61F 2/06

(54) **Rapid exchange emboli capture guidewire system**
Führungsdrahtsystem zum Abfangen von Emboli mit schnellem Austausch
Système de fil guide de capture des emboles d'échanges rapides

(30) Priority: 03.02.2006 US 347131
(43) Date of publication of application: 08.08.2007
(73) Proprietor: Cordis Corporation, Miami Lakes, FL 33014 (US)
(72) Inventor: Diaz, Pedro, Pembroke Pines, FL 33029 (US); Johnson, Kirk L., Davie, FL 33326 (US)
(74) Representative: Belcher, Simon James

(56) References cited:
- WO-A-99/16382
- US-A1- 2002 115 942
- US-A1- 2002 161 389
- US-A1- 2003 130 685
- US-A1- 2005 004 595

## Description

The present invention relates to wire guided devices used in a blood vessel or other lumen in a patient's body. More particularly, the present invention relates to a rapid exchange emboli capture guidewire system that reduces embolization during wire guided procedures.

During catheterization of a patient for angioplasty treatment or stenting procedures for vascular stenosis, a guide wire is directed through a patient's blood vessel to the site of interest. For example, the physician may wish to utilize a balloon catheter in order to enlarge a partially obstructed blood vessel at a certain location in the patient's vascular system. To do this, the physician utilizes a guide wire which is directed through the patient's vascular system to the particular site for balloon catheterization. Various medical devices are percutaneously inserted into the patient's blood vessel utilizing the guide wire. The balloon catheter, for example, is mounted at the distal end of an elongated tube. The guide wire is placed in the lumen of the balloon catheter tube such that the balloon catheter can be threaded over the guide wire, through the vascular system and placed at the site of interest by following the guide wire.

In order to enlarge a partially obstructed blood vessel, a physician may use various surgical techniques and biomedical devices or tools including balloon catheters, scrapers or other known medical devices. However, the utilization of these devices sometimes results in a release of an embolus (embolic material) which is an abnormal particle circulating in the blood, such as plaque and blood clots, that may move from the treatment site through a vein or artery, thus compromising the flow of blood at a location downstream. In order to reduce complications arising from dislodged emboli, physicians sometime utilize filters disposed downstream of the treatment site. As used herein the term "downstream" refers to an item that is spaced a distance apart from a referenced item and in the direction of blood flow through the blood vessel.

Frequently, the same guidewire used to carry the filter is also used to guide various catheters to and from the treatment site. For example, during angioplasty or stenting, various catheters are typically exchanged over the guidewire. When catheters are exchanged during a procedure, inadvertent and undesirable wire movement may occur. Such inadvertent movement can cause the filter to move into the treatment area requiring re-deployment. Worse, excessive movement can retract a filter proximally (upstream), where it could potentially become entangled in a deployed stent or inadvertently moved into a position where it can no longer protect the vessels it was deployed to protect.

Similar problems are encountered during a user's steering of catheter-sheathed device during inter-lumen procedures. Steering a sheathed device poses its own challenges in controlling the device relative the guidewire because a user manipulates the sheath as opposed to the wire during deployment and may cause accidental displacement of a device such as an embolic filter. Also, in some vessels, when guide catheters are repositioned, the filter tends to move within the vessels. This is undesirable not only because the vessel may be damaged by such movement, but also because captured emboli may become freed or new emboli released upstream of the filter.

US-A-2005/004595 discusses an embolic filtering device. A delivery sheath includes a shaft portion with a split seam channel or slit. This allows the delivery sheath to be peeled away from a guide wire after the embolic filtering device has been deployed. A torque handle can be mounted directly to the guidewire and has a side port through which the delivery sheath can be retracted to peel away from the guidewire.

US-A-2002/0115942 relates to an emboli capture device. In one embodiment a sleeve is splittable to allow its removal from a guidewire.

US-A-2003/0130685 discusses an emboli capture system. In one embodiment a shaft includes a predefined slit or score so that it can be peeled away.

Accordingly, there remains a long-felt need in the art for a guidewire method and system that improves a user's control during catheterization procedures. Also, as will be appreciated by one of ordinary skill in the art, the drawbacks of wire-guided systems are not limited to inadvertent movement during steering, deployment or catheter exchange. For example, the length of the guidewire necessary for these procedures and the time it takes to remove delivery catheter sheaths during deployment are directly correlated. There remains a continuing long-felt need in the art for a guidewire method and system that affords the use of shorter guide wires. Likewise, there remains a need in the art for a guidewire method and system that optimizes capture times in removing deployed devices through the deployment and retrieval of capture sheaths.

Various embodiments of the present invention offer advantageous features that may overcome the drawbacks discussed above and offer new advantages as well.

An objective of the invention is to provide an improved guidewire system for use in catheterization procedures. A related object of the invention is to provide a guidewire system that allows for improved steering of inter-lumen devices. Another object of the invention is to provide a guidewire system that allows for improved control of the core wire during catheterization procedures. An advantageous feature of at least one embodiment of the invention is the improved prevention of accidental displacement of emobolic filters and the like during catheterization procedures.

It is another object of the invention to provide a guidewire system having a rapid exchange design. It is a related object of the invention to provide a guidewire system that allows for quick removal of a delivery sheath to expose the corewire during catheterization procedures. Another related object of the invention to provide a guidewire system that allows for more rapid capture procedure during catheterization procedures. An advantageous feature at least one embodiment of the invention is the ability to use shorter core wires during catheterization procedures.

It is another object of the invention to provide a guidewire system comprising a rapid exchange emboli capture guidewire system that reduces macro and micro-embolization during intra-lumen procedures such as angioplasty treatment and stenting procedures of vascular stenosis.

According to the present invention there is provided a delivery sheath as defined in the appended claim 1.

According to another aspect of the invention, there is provided an emboli capture guidewire system as defined in appended claim 9. It may comprise a corewire having distal floppy region, which may act as a stop, and an embolic filter collapsible and deployable on said corewire. The corewire is positioned in an area of a site to be treated in a manner that allows the filter (which is disposed adjacent and proximal of the distal floppy region) to be deployed in an area distal the treatment area for capturing emboli released during treatment procedures.

The emboli capture system is preferably assembled inside a delivery sheath prior to closing the lesion. The distal end of the delivery sheath collapses the embolic filter to lower its profile and facilitate crossing the target lesion for positioning the filter. The delivery sheath is configured to allow peeling of the sheath from the corewire. In a preferred embodiment, the delivery sheath includes a sheath slit located in the proximal shaft and preferably also a small portion of the distal shaft. The sheath slit provides an exit for egression of the adjacent areas of the corewire from the delivery sheath during peeling. Use of a peelable sheath permits quick removal of the delivery sheath to expose the corewire. Use of a peelable sheath according to the invention allows the user to gain control of the wire to prevent accidental displacement of the filter and also allows for shorter wire lengths to be used during catheterization procedures. As will be appreciated, the longer the length of wire used, the more time it takes to remove sheaths and devices from the corewire.

According to another aspect of the invention, a torque device is used to clamp on the corewire to facilitate steering of the emboli system to access the lesion. In a preferred embodiment, the torque device is designed with a recess that allows a peel-initiator handle to dock inside a nub of the torque device. Preferably, the torque device clamps on the corewire but not on the peel-initiator handle to prevent steering of the delivery sheath.

The filter is deployed by pulling the delivery sheath back. According to an advantageous feature of the invention, once the filter is deployed, the delivery sheath can then be peeled away from the corewire by pulling on the peel-initiator handle. Preferably, the peeling of the sheath is facilitated by the delivery sheath slit defined in the proximal shaft and a portion of the distal shaft. In a preferred embodiment, the distal shaft comprises a different color than the proximal shaft to indicate to the user the area where the end of the slit is located. The distal end of the slit is closer to the distal end than to the peel-initiator handle. As will be appreciated, once the delivery sheath is removed from the corewire, a PTA, PTCA, or stent delvery system can be loaded over the corewire to perform the angioplasty or stening procedure.

Upon completion of the angioplasty, stenting or other treatment procedure, a capture sheath is used to gain access to the filter and retrieve it. According to an aspect of the invention, a capture sheath having a side-port opening is provided. In operation, the distal tip of the capture sheath is configured to engage and collapse the filter to secure the captured emboli and reduce the filter profile for retrieval from the lumen. According to this aspect of the invention, the distal end of the capture sheath rides over the corewire with the corewire exiting the side-port. This configuration of the capture sheath and corewire accelerates the capture procedure.

In a preferred embodiment, the side port of the sheath is located closer to the distal tip than to a proximal handle used to manipulate movement of the sheath. The proximal shaft area is also made more rigid than the distal shaft of the capture sheath to facilitate pushing of the sheath. This configuration further accelerates the speed and alacrity of the capture procedure.

The system of the present invention can be used in a method of performing a catheterization procedure. The method comprises: inserting an emboli capture guidewire system into a delivery sheath and delivering said emboli capture guidewire system to a downstream side of a lesion to be treated using a torque device clamped on a corewire of said system; removing said torque device and peeling away said delivery sheath from an area of said wire via a longitudinal exit spanning from a proximal end of said sheath to an area in said distal end of said sheath; proximally withdrawing the remainder of said delivery sheath from said corewire; inserting a treatment system over the corewire to the lesion and performing the treatment procedure; removing the treatment system; inserting a capture sheath and capturing said distal protection device; and removing the capture sheath and corewire.

Preferably, the emboli capture system comprises an emboli filter and the treatment system comprises a balloon catheter angioplasty system or a stenting system.

Embodiments of the invention will now be described by way of example with reference to the accompanying drawings where like reference numerals indicate identical or functionally similar elements, and in which:
Figure 1 illustrates an exemplary guidewire filter system for use in connection with the present invention.
   Figure 2 illustrates an embodiment of a delivery sheath according to the invention.
Figure 3 illustrates an embodiment of a delivery sheath loaded with a guidewire filter system for deployment according to the invention.
Figure 4 is a cross-sectional view of Figure 3 taken along lines A-A.
Figure 5 is a cross-sectional view of Figure 3 taken along lines B-B.
Figure 6 illustrates an embodiment of a dellvery sheath being removed from the corewire of a guidewire filter system after deployment of the filter.
Figure 7 illustrates an embodiment of a capture sheath according to the invention.
Figure 8 illustrates an embodiment of a capture sheath in the process of retrieving a deployed filter of a guidewire filter system.
Figure 9 illustrates a torque device for steering a guidewire system according to the invention.
Figure 10 illustrates alternative configurations of peelable sheath and torque clamping locations according to the invention.

While the present invention will be described in connection with an embolic filter system for use in catheterization procedures, one of ordinary skill in the art will recognize the universality of the advantageous aspects of the invention, including the applicability in the deployment and capture of devices in any intra-lumen procedure.

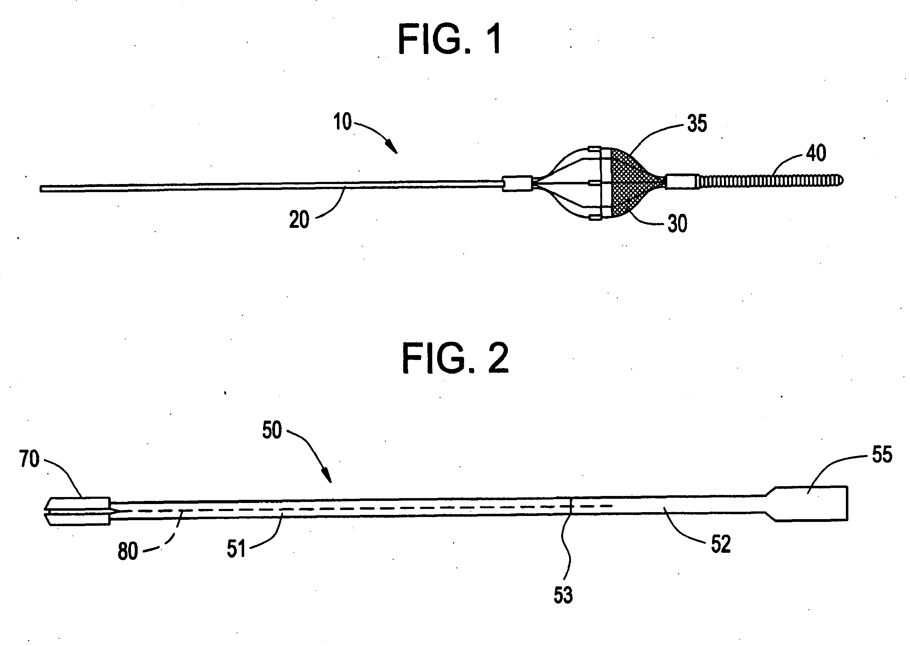

The various figures depict varying aspects of preferred embodiments of a rapid exchange emboli capture guidewire system according to the invention. A rapid exchange emboli capture system configured according to the invention may be useful in reducing macro and micro-embolization during angioplasty treatment and stenting procedures of vascular stenosis. As depicted in Fig. 1, the rapid exchange emboli capture guidewire system includes a guidewire filter system 10 that contains an embolic filter 30 positioned proximal to a distal floppy region 40 of a corewire 20.

The embolic filter 30 is configured to be delivered to a site downstream to the lesion being treated to capture particles that break loose during a PTA, PTCA, or stenting procedure. To achieve this end, the embolic filter 30 is preferably made out of nitinol and includes a polymeric membrane 35 on its distal end with holes 36 to facilitate filtration of blood. Hole sizes preferably range from 50 to 200 µm (50 to 200 microns) to allow for the passage of blood and the capture of emboli. The exact configuration of the filter is not essential to the invention and any suitable filter for the intra-lumen procedure being performed is deemed within the scope of the invention. Likewise, while a filter is used in connection with the described embodiments, it should be understood that any suitable distal protection device adapted to be deployed in a lumen or vessel of a patient may be used in connection with the present invention

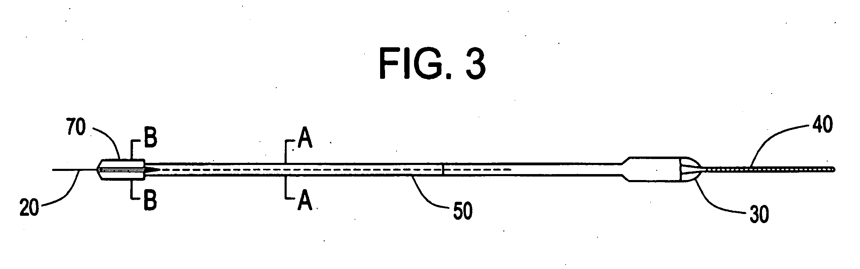

Turning to Fig. 2, a delivery sheath 50 is provided for facilitating delivery of the guidewire filter system 10 into its proper position. The guidewire filter system 1 is assembled inside delivery sheath 50 prior to closing the lesion. To achieve this end, the distal end 55 of the delivery sheath is configured to collapse the embolic filter 30 to lower its profile and facilitate crossing the target lesion. Figure 3 depicts a delivery sheath 50 loaded with a guidewire filter system 10 during the delivery process prior to deployment of the filter.

As shown in Figs. 2 and 3, the delivery sheath 50 is also provided with a peel-initiator handle 70 and a longitudinal sheath slit 80, the significance of which will be explained herein below. The sheath slit 80 preferably spans an area along a length of the proximal shaft 51 of the delivery sheath 50 and a portion of the distal shaft 52. Line 53 in Figs. 2 and 3 indicates an approximate demarcation of the division between the proximal shaft portion 51 and the distal shaft portion 52. Preferably the area of distal shaft 52 adjacent the demarcation 53 comprises a color or other visually distinguishable feature than the area adjacent the demarcation 53 on the proximal shaft 51 to provide an indication to a user where the sheath slit 80 ends.

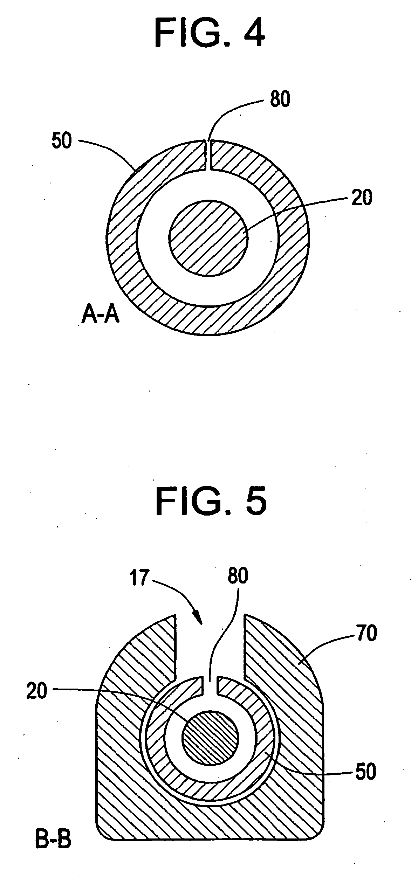

Figure 4 is a cross-section view taken along lines A-A of Fig. 3 to highlight the relative size of the sheath slit 80 compared to the diameter of corewire 20. The slit 80 is preferably sized to hold corewire 20 from accidental or inadvertent removal. At least some level of intentional friction or pressure is required to cause corewire 20 to protrude through slit 80. While sheath slit 80 is shown as a continuous open fissure along its length, it is equally feasible to provide a slit comprising perforations or providing a slit having a breakable membrane. Any suitable method of retaining the corewire 20 until its desired to laterally remove a portion of the delivery sheath 50 is contemplated by the present invention.

Figure 5 is a cross-sectional view taken along lines B-B of Fig. 3 to illustrate the orientation of the guidewire 20, the delivery sheath slit 80, and the peel-initiator handle 70. As depicted, peel-initiator handle 70 cradles delivery sheath 50 (and the corewire housed therein) in a manner exposing slit 80 to an open area 17 through which corewire 20 may laterally pass therethrough upon exiting the slit 80 while the delivery sheath 50 remains behind with the handle 70.

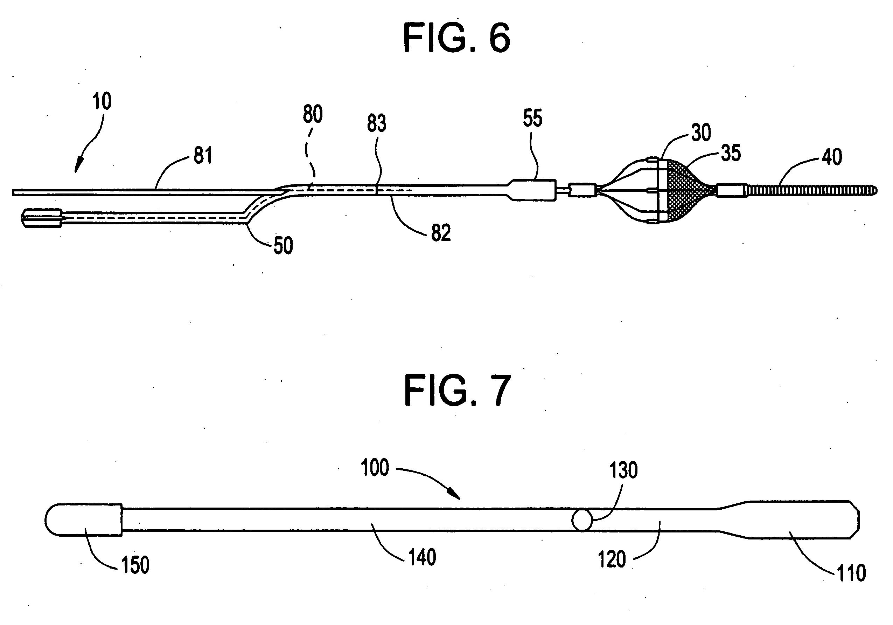

Turning back to Fig. 3, the loaded filter is steered into an area distal the lesion being treated in order to be deployed in a position to capture emboli released into the blood during the lesion closing treatment. As shown in Fig. 6, the filter 30 is deployed by pulling the delivery sheath 50 back to reveal filter 30 from the distal end 55. Once the filter 30 is deployed, the delivery sheath 50 provides no other use in the treatment being performed. Accordingly, the delivery sheath 50 must be removed from the corewire 20 to free up the corewire for placement and advancement of catheters housing treating devices, such as stents or angioplasty balloons, to the lesion area.

To speed the removal of the delivery sheath 50 from the system, the peel-initiator handle 70 and sheath slit 80 come into play. In short, the delivery sheath is "peeled away" from the corewire 20 as it is shown to be in the process of in Fig. 6.

The peeling away of the delivery sheath 50 is initiated by pulling on the peel-initiator handle 70. Actuating the handle 70 allows the delivery sheath 50 to be removed from around the corewire 20 by using sheath slit 80 as an exit that will not overly disturb the corewire 20 as the sheath 50 is removed. The differentiated coloring of the proximal shaft 51 and the distal shaft 52 also aids the non-disturbing removal of the sheath 50 by indicating to a user when the end portion of the slit 80 and/or distal end is being neared. Preferably, the distal end of the slit is preferably closer to the distal end than the peel-initiator handle. Again, at this time, the delivery sheath is removed from the system freeing up the corewire for loading of a PTA, PTCA, or stent delivery system to perform angioplasty or stenting procedures.

At the conclusion of the treating of the stenosis by angioplasty or stenting, the emboli filter must be removed. In order to close the filter 30 and retain any captured emboli, a capture sheath 100 is used. A presently preferred rapid capture sheath 100 according to the invention is depicted in Fig. 7. As shown, the capture sheath 100 includes a distal tip 110 on its distal end 120, a proximal handle 150 on its proximal end 140, and a side-port 130 disposed between the distal tip 110 and handle 150. The side-port allows the distal end 120 of the capture sheath 100 to ride on the corewire 20. In other words, the capture sheath is threaded onto the corewire 20 in a manner that the corewire passes through the center of the distal tip, through a portion of the shaft and out of the sheath via the side-port 130. As will be appreciated, the use of a side port saves time in not having to thread the corewire down the entire length of the catheter.

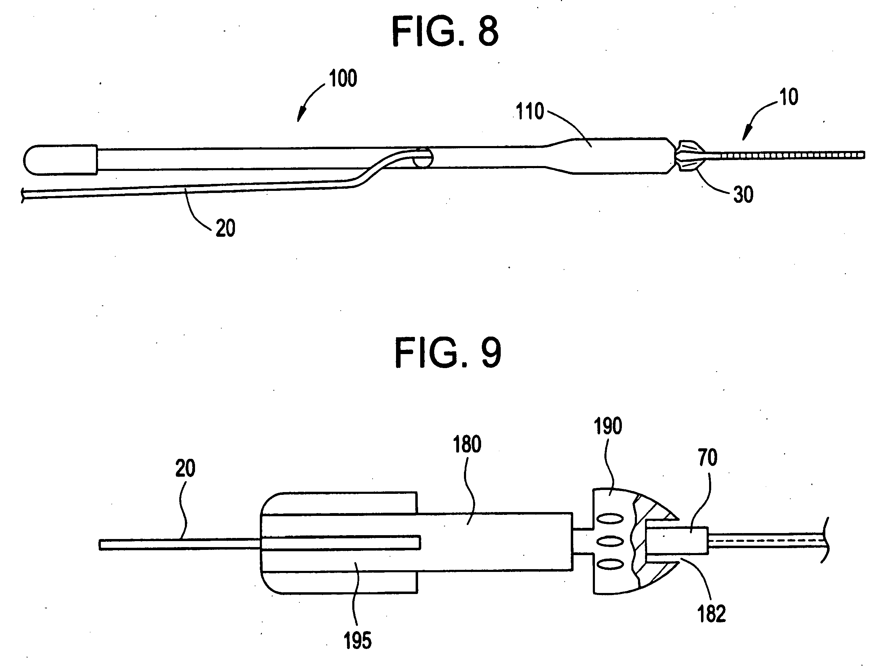

As best shown in Fig. 8, once threaded onto the corewire, the capture sheath 100 is advanced with use of handle 150 until distal tip 110 swallows filter 30 in a manner collapsing its profile and retaining any captured emboli inside filter membrane35. To facilitate advancement of the capture sheath 100, the proximal shaft portion is more rigid than the distal shaft portion. Increased rigidity allows for easier pushing of the sheath into an operative position for capturing and retrieving the filter, again, another possible timesaving measure.

Another advantageous aspect of the invention is depicted in Fig. 9. In various preferred embodiments of the invention, a torque device 180 is used to steer the system into its proper place. As shown in Fig. 9, the torque device 180 may be used to steer the embolic filter system 10 into its proper place for delivery. The torque device 180 according to this embodiment comprises a distal nub portion 190 and a proximal body portion 195. The inner wall of the torque device 180 defines a passageway for accepting the corewire 20 and is clampable thereon. The torque device 180 includes a recess 182 in the nub 190 for receiving the peel-initiator handle 70 of the delivery sheath 50.

In this embodiment, the torque device 180 is used to facilitate steering of the system to access the lesion. The provision of a recess 182 in the nub 190 allows for the peel-initiator handle 70 to dock during deployment. This configuration allows the torque device 180 to clamp onto the corewire 20 but not onto the peel-initiator handle 70. Clamping onto the corewire 20 without clamping onto the peel-initiator handle 70 provides for steering of the filter system 10 without steering of the delivery sheath 50. The exact configuration of the torque device is not essential to the invention. One of ordinary skill in the art armed with the present specification is capable of adapting a steering device for use in the invention. The steering device preferably clamps only on the corewire for the reasons cited herein, but alternate arrangements are within the scope of the invention.

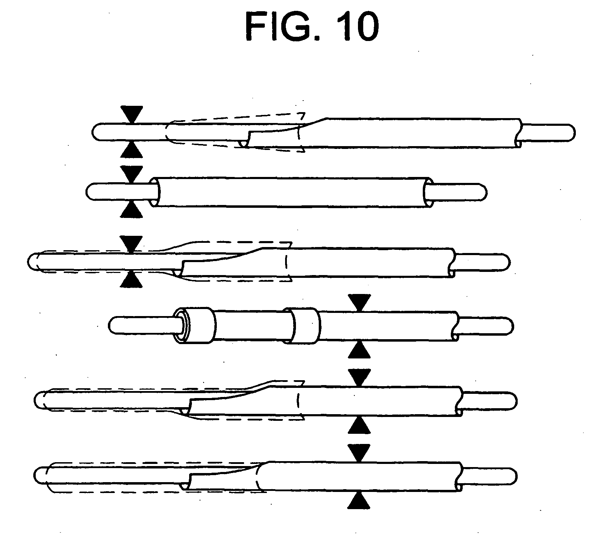

In this vein, alternate locations of clamping as well as alternate methods of initiating peeling of the delivery sheath are depicted in Fig. 10 for illustrative purposes to demonstrate the expanse of alternate embodiments employing various adaptations of some of the advantageous features of the invention. In some alternate embodiments, the proximal end of the delivery sheath is cut away in a manner to allow the user to initiate the peeling away process without the need for a peel-initiator handle. For example, the sheath may be provided with half the wall cut away at the slit side. A cut-away provides an area a user can manipulate to start the peeling process.

In embodiments having a user initiated peel away process, such as those using a cut-away, another advantageous aspect of the invention is the possible provision of an end protector in a location on the cut-away over the wire. The end protector serves to prevent pre-initiation of the peeling process and to prevent resultant premature filter deployment.

An exemplary method of using a rapid exchange emboli capture guidewire system according to the invention comprises the steps of inserting a filter loaded delivery sheath to the distal side of a lesion being treated. The filter system is preferably steered using a torque device that clamps onto the corewire while leaving the delivery sheath free. The filter is then deployed by pulling back on the delivery sheath and holding the guidewire stationary. Pulling back the delivery sheath allows the struts of the filter to expand distal of the lesion and capture any emboli freed during the treatment process. The torque if used is removed after filter deployment. The delivery sheath is then peeled away from the corewire, preferably aided by the peel-initiator hub separating the delivery sheath from the corewire.

The peeling process is continued and the deployment sheath is withdrawn to the end of the sheath slit. The remainder of the distal shaft is removed over the guidewire proximally. A balloon catheter or a stent delivery system is inserted over the guidewire to the lesion and the angioplasty or stenting procedure is performed according to methods known in the art. The angioplasty catheter or stent delivery system is removed. At this time a rapid exchange capture sheath having a side-port is used to capture the filter and collapse it into a lower profile for removal while retaining any captured emboli. Finally, while holding both the proximal shaft or handle of the capture sheath and the guidewire firmly, the two are pulled back to retrieve the system from the patient's body.

## Claims

1. A delivery sheath (50) for use in catheterization processes comprising:
a lumen having a distal end (55), a proximal end, and a sidewall defining an interior channel configured to allow a wire to longitudinally transverse through said lumen; wherein said sidewall further includes a longitudinal exit (80) along a length of said lumen for allowing a portion of a wire (20) disposed therein to pass laterally therethrough; and
**characterised by**:
a peel-initiator handle (70) adapted to allow a user to initiate the removal of said sheath (50) from an area of a wire (20) disposed in said channel via said longitudinal exit (80), and wherein said peel-initiator handle exposes said longitudinal exit (80) to an open area (17) for allowing said wire (20) to laterally pass therethrough.

2. The delivery sheath of claim 1, further comprising a distal bulb defined by said distal end for housing a wire-guided device to be delivered.

3. The delivery sheath of claim 1, wherein said longitudinal exit (80) extends from said proximal end into a portion of said distal end (55).

4. The delivery sheath of claim 3, wherein said longitudinal exit comprises a slit (80) sized to retain a wire (20) disposed therein while allowing said sheath (50) to be peeled away from said wire (20).

5. The delivery sheath of claim 3, wherein said longitudinal exit comprises a series of perforations adapted to allow said sheath (50) to be peeled away from a wire (20) disposed in said channel for the length transversed by said perforations.

6. The delivery sheath of claim 3, wherein said longitudinal exit comprises a longitudinal scored area adapted to allow said sheath (50) to be peeled away from a wire (20) disposed in said channel for the length transversed by said scored area.

7. The delivery sheath of claim 3, wherein said exit comprises a channel having a frangible membrane adapted to retain said wire (20) while allowing said sheath (50) to be peeled away from a length of said wire (20) adjacent said channel.

8. The delivery sheath of claim 3, wherein the distal portion (52) of the delivery sheath (50) comprises a different color or other visually distringuishable feature than the proximal portion (51) of the delivery sheath to indicate to a user the area where the end of the longitudinal exit (80) is located.

9. An emboli capture guidewire system comprising:
a wire-guided distal protective device (30);
a delivery sheath according to any one of the preceding claims.

10. The system of claim 9, wherein said distal protective device comprises an emboli filter (30).

11. The system of claim 9, further comprising a torque device (180) for steering said wire-guide distal protective device.

12. The system of claim 11, wherein said torque device (180) clamps onto said wire without clamping onto said peel-initiator handle (70), thereby allowing said wire-guided device to be steered without steering of said delivery sheath (50).

13. The system of claim 9, further comprising a capture sheath (100) for retrieving said distal protection device; said capture sheath (100) including a side-port (130) that allows said capture sheath (100) to be thread onto said wire (20) in a manner that said wire (20) passes into said capture sheath (100) through a distal opening and exits said side-port.

14. The system of claim 13, wherein said capture sheath (100) includes a distal bulb for collapsing and holding said distal protection device; and
a rigid proximal portion that facilitates maneuvering said capture sheath (100).

## Patentansprüche

1. Zuführungshüllrohr (50) zur Benutzung in Katheterisierungseingreiffen, das Folgendes aufweist:
ein Lumen mit einem distalen Ende (55), einem proximalen Ende und einer Seitenwand, die einen inneren Kanal definiert, der dafür konfiguriert ist, einem Draht zu ermöglichen, das Lumen longitudinal zu durchqueren, wobei die Seitenwand weiterhin einen longitudinalen Ausgang (80) entlang einer Länge des Lumens aufweist, um es einem Abschnitt eines Drahtes (20), der darin angeordnet ist, zu ermöglichen, seitlich durch diesen hindurch zu treten, und **gekennzeichnet ist durch**:
ein Peel-Initiator-Handstück (70), das dafür eingerichtet ist, einem Benutzer zu ermöglichen, das Entfernen des Hüllrohrs (50) über einen Bereich eines Drahtes (20), der in dem Kanal angeordnet ist, **durch** den longitudinalen Ausgang (80) zu initiieren und
wobei das Peel-Initiator-Handstück den longitudinalen Ausgang (80) zu einem offenen Bereich (17) aufdeckt, um zu ermöglichen, dass er Draht (20) seitlich **durch** diesen hindurchtritt.

2. Zuführungshüllrohr nach Anspruch 1, das weiterhin einen distalen Wulst umfasst, der durch das distale Ende definiert ist, um eine drahtgeführte Vorrichtung, die zugeführt werden soll, aufzunehmen.

3. Zuführungshüllrohr nach Anspruch 1, bei welchem der longitudinale Ausgang (80) sich von dem proximalen Ende in einen Abschnitt des distalen Endes (55) erstreckt.

4. Zuführungshüllrohr nach Anspruch 3, bei welchem der longitudinale Ausgang einen Schlitz (80) umfasst, der dafür bemessen ist, einen Draht (20), der darin angeordnet ist, zurückzuhalten, während ermöglicht wird, das Hüllrohr (50) von dem Draht (20) abzuziehen.

5. Zuführungshüllrohr nach Anspruch 3, bei welchem der longitudinale Ausgang eine Abfolge von Perforationen umfasst, die dafür eingerichtet sind, zu ermöglichen, das Hüllrohr (50) von einem Draht (20), der in dem Kanal angeordnet ist, über die Länge abzuziehen, die von den Perforationen durchquert wird.

6. Zuführungshüllrohr nach Anspruch 3, bei welchem der longitudinale Ausgang einen angeritzten Bereich umfasst, der dafür eingerichtet ist, zu ermöglichen, das Hüllrohr (50) von einem Draht (20), der in dem Kanal angeordnet ist, über die Länge abzuziehen, die von dem angeritzten Bereich überquert wird.

7. Zuführungshüllrohr nach Anspruch 3, bei welchem der Ausgang einen Kanal umfasst mit einer brüchigen Membran, die dafür eingerichtet ist, den Draht (20) zurückzuhalten, während ermöglicht wird, das Hüllrohr (50) von einer Länge des Drahtes (20), die an den Kanal angrenzt, abzuziehen.

8. Zuführungshüllrohr nach Anspruch 3, wobei der distale Abschnitt (52) des Zuführungshüllrohrs (50) eine andere Farbe oder ein anderes visuell unterscheidbares Merkmal aufweist als der proximale Abschnitt (51) des Zuführungshüllrohrs, um einen Benutzer den Bereich anzuzeigen, in dem sich das Ende des longitudinalen Ausgangs (80) befindet.

9. Embolieeinrangführungsdrahtsystem, das folgendes umfasst:
eine drahtgeführte distale Schutzvorrichtung (30);
ein Zuführungshüllrohr nach einem der vorhergehenden Ansprüche.

10. System nach Anspruch 9, bei welchem die distale Schutzvorrichtung einen Emboliefilter (30) umfasst.

11. System nach Anspruch 9, das weiterhin eine Verdrehvorrichtung (180) zum Lenken der drahtgeführten distalen Schutzvorrichtung umfasst.

12. System nach Anspruch 11, bei welchem die Verdrehvorrichtung (180) auf dem Draht klemmt, ohne auf dem Peel-Initiator-Handstück (70) zu klemmen, wodurch ermöglicht wird, die drahtgeführte Vorrichtung zu lenken, ohne das Zuführungshüllrohr (50) zu lenken.

13. System nach Anspruch 9, das weiterhin ein Einfanghüllrohr (100) zum Erfassen der distalen Schutzvorrichtung aufweist, wobei das Einfanghüllrohr (100) einen Seitenanschluss (130) aufweist, der es ermöglicht, das Einfanghüllrohr (100) auf einen Draht (20) in einer Weise aufzufädeln, dass der Draht (20) in das Einfanghüllrohr (100) durch eine distale Öffnung passt und den Seitenanschluss verlässt.

14. System nach Anspruch 13, bei welchem das Einfanghüllrohr (100) einen distalen Wulst zum Zusammenziehen und Halten der distalen Schutzvorrichtung umfasst und einen steifen proximalen Abschnitt, der das Manövrieren des Einfanghüllrohrs (100) unterstützt.

## Revendications

1. Gaine de distribution (50) à utiliser dans les processus de cathétérisation comprenant :
◆ une lumière ayant une extrémité distale (55), une extrémité proximale, et une paroi latérale définissant un canal intérieur configuré pour permettre à un fil de traverser longitudinalement ladite lumière ; dans laquelle ladite paroi latérale comprend en outre une sortie longitudinale (80) sur une longueur de ladite lumière afin de permettre à une portion d'un fil (20) disposé à l'intérieur de passer latéralement à travers ; et **caractérisée par** :
◆ une poignée de démarrage de détachement (70) adaptée pour permettre à un utilisateur de commencer l'enlèvement de ladite gaine (50) d'une zone d'un fil (20) disposé dans ledit canal via ladite sortie longitudinale (80) et dans laquelle ladite poignée de démarrage de détachement expose ladite sortie longitudinale (80) à une zone ouverte (17) pour permettre audit fil (20) de passer latéralement à travers.

2. Gaine de distribution selon la revendication 1, comprenant en outre un bulbe distal défini par ladite extrémité distale pour abriter un dispositif à guide-fil à distribuer.

3. Gaine de distribution selon la revendication 1, dans laquelle ladite sortie longitudinale (80) s'étend depuis ladite extrémité proximale dans une portion de ladite extrémité distale (55).

4. Gaine de distribution selon la revendication 3, dans laquelle ladite sortie longitudinale comprend une fente (80) dimensionnée pour retenir un fil (20) disposé à l'intérieur tout en permettant à ladite gaine (50) d'être décollée dudit fil (20).

5. Gaine de distribution selon la revendication 3, dans laquelle ladite sortie longitudinale comprend une série de perforations adaptées pour permettre à ladite gaine (50) d'être décollée d'un fil (20) disposé dans ledit canal sur la longueur traversée par lesdites perforations.

6. Gaine de distribution selon la revendication 3, dans laquelle ladite sortie longitudinale comprend une zone perforée longitudinale adaptée pour permettre à ladite gaine (50) d'être décollée d'un fil (20) disposé dans ledit canal sur la longueur traversée par ladite zone perforée.

7. Gaine de distribution selon la revendication 3, dans laquelle ladite sortie comprend un canal ayant une membrane pouvant se rompre adaptée pour retenir ledit fil (20) tout en permettant à ladite gaine (50) d'être décollée d'une longueur dudit fil (20) adjacente audit canal.

8. Gaine de distribution selon la revendication 3, dans laquelle la portion distale (52) de la gaine de distribution (50) comprend une couleur différente ou une autre caractéristique pouvant se distinguer visuellement de la portion proximale (51) de la gaine de distribution, afin d'indiquer à un utilisateur la zone où l'extrémité de la sortie longitudinale (80) est située.

9. Système guide-fil à capture d'emboles comprenant :
◆ un dispositif de protection distal àguide-fil (30) ;
◆ une gaine de distribution selon l'une quelconque des revendications précédentes.

10. Système selon la revendication 9, dans lequel ledit dispositif de protection distal comprend un filtre à emboles (30).

11. Système selon la revendication 9, comprenant en outre un dispositif à couple (180) pour manoeuvrer ledit dispositif de protection distal à guide-fil.

12. Système selon la revendication 11, dans lequel ledit dispositif à couple (180) se fixe sur ledit fil sans se fixer sur ladite poignée de démarrage de détachement (70) en permettant ainsi de manoeuvrer ledit dispositif à guide-fil sans manoeuvrer ladite gaine de distribution (50).

13. Système selon la revendication 9, comprenant en outre une gaine de capture (100) pour récupérer ledit dispositif de protection distal ; ladite gaine de capture (100) comprenant un orifice latéral (130) qui permet à ladite gaine de capture (100) d'être vissée sur ledit fil (20) de manière à ce que ledit fil (20) passe dans ladite gaine de capture (100) à travers une ouverture distale et sorte par ledit orifice latéral.

14. Système selon la revendication 13, dans lequel ladite gaine de capture (100) comprend un bulbe distal pour replier et maintenir ledit dispositif de protection distal ; et une portion proximale rigide qui facilite la manoeuvre de ladite gaine de capture (100).
